# EUROPEAN PATENT APPLICATION

(11) **EP 2 526 889 A1**
(43) Date of publication of application: **28.11.2012**
(21) Application number: 12003962.3
(22) Date of filing: 21.05.2012
(51) Int. Cl.: A61B 19/08, F16B 2/22, F16B 21/06, A61B 6/00

(54) **A clip for a disposable cover**

(30) Priority: 21.05.2011 GB 201108628
(71) Applicant: Kuester Medical Limited Centre of Innovation, Oxfordshire OX5 1PF (GB)
(72) Inventor: Rye, Martin Cee, Twyford, Banbury Oxfordshire OX17 3JQ (GB)
(74) Representative: Franks & Co (South) Limited

(57) **Abstract**

A mounting clip for holding a sterile cover means in place on an arm of a medical apparatus, the mounting clip being a substantially U-shaped clip formed from a resilient material allowing the ends of the mounting clip to flex towards and away from each other to facilitate gripping the arm. The inner end portions of the mounting clip have a leg pivoted thereto which in use engages the surface of the arm. A cover means for sterilizing an arm of a medical apparatus comprises a drape and a plurality of the mounting clips spaced along the cover means.

## Description

### Field of the Invention

The present invention relates to clips for holding sterile covers for medical instruments, and in particular to clips for use with covers for the C-arms of X-ray machines and also to the assembled covers.

### Background of the Invention

One method of keeping medical equipment sterile is to use a throw-away sterile cover which is wrapped around the medical equipment. The cover is used only once and is thrown away after use.

US Patent No. 5,506,882 discloses a C-arm for an X-ray machine in which the arm is provided with grooves formed in the sidewalls of the arm and clips which hold the cover in place and engage in the grooves in the arm. A major disadvantage of this arrangement is that the cover can only be attached to C-arms which have to be made with grooves to receive the clips.

This problem has been overcome by the cover and clips disclosed in International Patent Publication No. WO 97/17035 A1 which discloses a sterile cover and clips suitable for use with C-arms without the provision of grooves. The cover is provided with U-shaped clips which resiliently grip onto the C-arm and the cover is mounted over the clips and held in place by fasteners formed from hinged end portions of the clip arms.

A disadvantage of this arrangement is that the clips must have suitable dimensions to ensure that the clips grip onto the C-arm. This limits the range of arms for which one clip size is suitable. Furthermore the arms of the clips tend to grip the C-arm through a point contact which means that the clips can easily slip from their desired locations.

The present invention seeks to provide a clip for a sterile cover which has an improved grip on the C-arm and is more universal in its applications.

### Summary of the Invention

According to a first aspect there is provided a mounting clip for holding a sterile cover means in place on an arm of a medical apparatus, the mounting clip being a substantially U-shaped clip formed from a resilient material allowing the ends of the clip to flex towards and away from each other to facilitate gripping the arm, **characterised in that** the inner end portions of the clip have a leg pivoted thereto which in use engages the surface of the arm.

The leg increases the surface contact of the clip with the arm of the medical apparatus and allows the clip to grip onto tapered or slightly inclined surfaces.

Preferably the clip has ends flaring outwards for easy attachment to said arm, such that the flared ends open out said clip when said clip is forcibly pressed over the C-arm and are resiliently biased to clasp said C-arm.

The legs are each pivoted to the clip at the point where the ends of the clips begin the flare outwards and preferably each leg extends on each side of the pivot point. Each leg may extend for substantially the full length of the respective flared end and the outer end of the leg may be accommodated within a recessed portion of its respective clip end.

The oppositely directed face of the clip may be formed with grooves therein which provide detents for cover retention clips that engage in the opposed grooves. Additionally or alternatively, the covers may be held in place at the ends of the mounting clip by retention clips that snap over detents on the outer surfaces of the flared end portions.

The mounting clip and retention clips may be formed from a suitable plastics material such as polyolefin e.g. polypropylene, or a polyamide

According to a second aspect there is provided a cover means for sterilizing an arm of medical equipment, and in particular the C-arms of X-ray machines, the cover means comprising a drape having dimensions adapted for covering three sides of said C-arm over the whole or part of its length; and a plurality of mounting clips according to the first aspect for mounting the drape to the C-arm and which are spaced along the cover means.

The mounting clips are dispersed along the cover means at desired intervals to conform with the shape and configuration of the arm.

The drape may be made of any suitable material, for example a suitable plastic film such as polyolefin, PET or PVC (polyvinyl chloride) film or a textile material for example cotton viscose.

The cover means may be sterilized and enclosed in a sterile cover for sterilizing the arm when said cover means is attached to the arm.

### Brief Description of the Drawings

For a better understanding of the invention and to show how the same may be carried into effect, there will now be described by way of example only, specific embodiments, methods and processes according to the present invention with reference to the accompanying drawings in which:
*Figure 1* is a plan view of a mounting clip according to the present invention,
*Figure 2* is an isometric view of the clip shown in *Figure 1* with the legs pivoted to a different condition,
*Figure 3* is a sectional view of a second clip being offered to a C-arm of an X-ray machine,
*Figure 4* shows the clip of *Figure 3* fully located on the C-arm,
*Figure 5* shows another embodiment of the invention, and
*Figure 6* shows a cover means according to a second aspect of the invention utilising clips as shown in *Figure 5**.*

### Detailed Description of the Embodiments

There will now be described by way of example a specific mode contemplated by the inventor. In the following description numerous specific details are set forth in order to provide a thorough understanding. It will be apparent however, to one skilled in the art, that the present invention may be practiced without limitation to these specific details. In other instances, well known methods and structures have not been described in detail so as not to unnecessarily obscure the description.

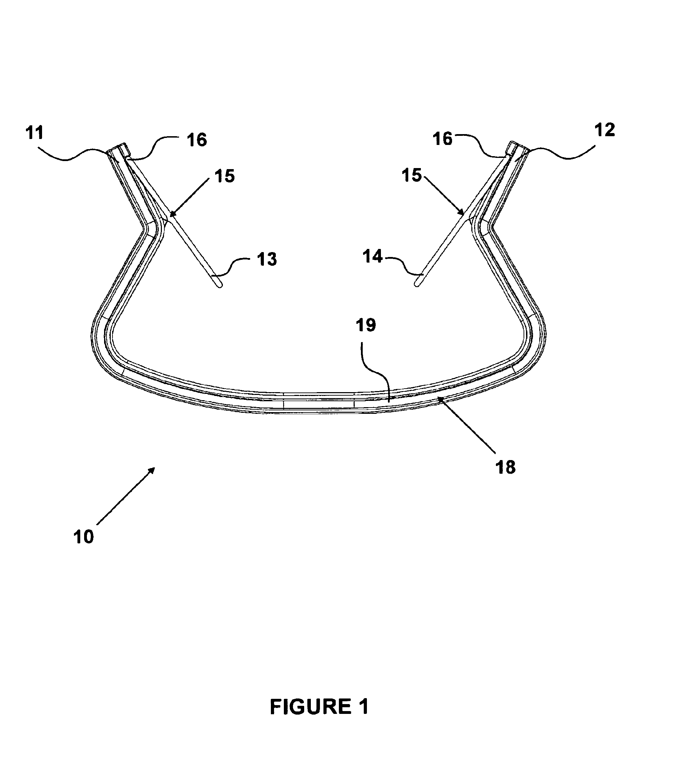

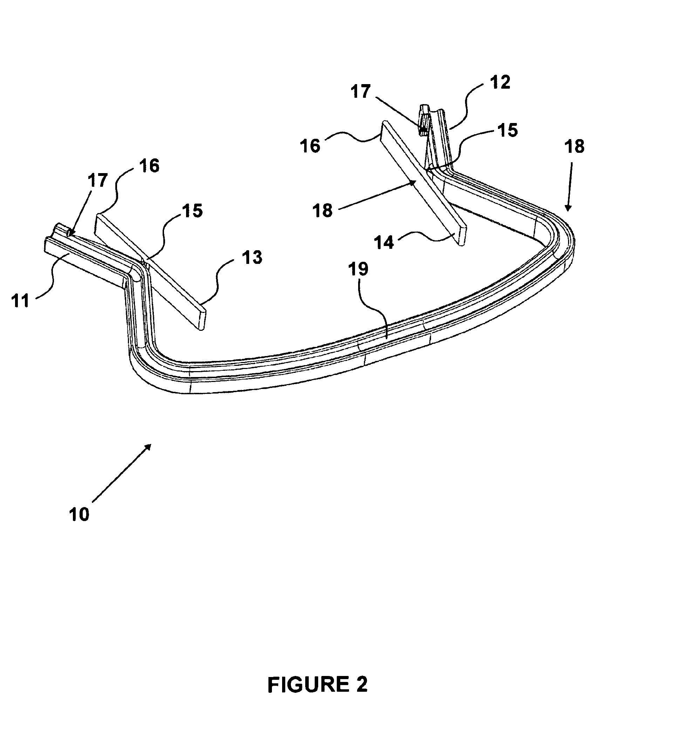

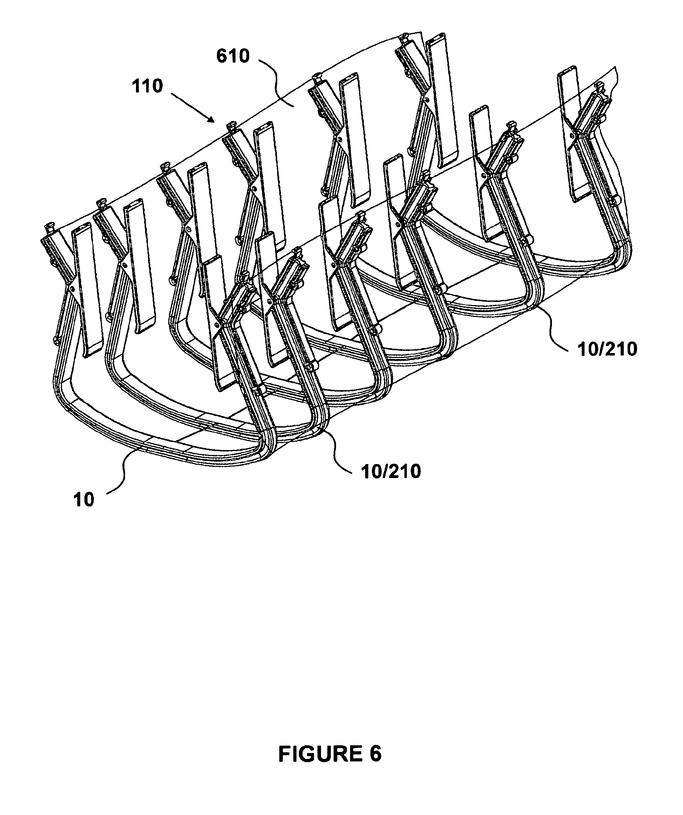

With reference to Figures 1 and 2, there is shown a mounting clip 10 for holding a sterile cover means 110 (see Figure 6) in place on an arm A of a medical apparatus. The mounting clip 10 is a substantially U-shaped clip formed from a resilient material allowing the ends of the clip to flex towards and away from each other to facilitate a clamping grip over the arm A. The clip is preferably moulded from a suitable plastics material for example polypropylene or a polyamide. The end portions 11,12 of the clip flare outwards for easy attachment to the arm A, such that the flared ends open out the clip when the clip is forcibly pressed over the arm A and are resiliently biased together to grip the arm.

A pair of contact legs 13 & 14 are pivoted one to each inner end portion of the clip. The contact legs 13 & 14 rotate about a respective pivot 15 located where the end portions of the clip begin to flare outwardly. Each contact leg 13 & 14 has a substantially flat inner surface 18 that in use sits against the arm A. The legs 13 & 14 extend both inwardly and outwardly relative to the pivot 15 and preferably each leg extends outwardly for substantially the full length of the respective flared end portion 11 or 12. The outer end 16 of each leg is accommodated within a recess 17 at its respective clip end as is shown in Figures 1 and 2. This provides for a smooth contact against the surface of the arm.

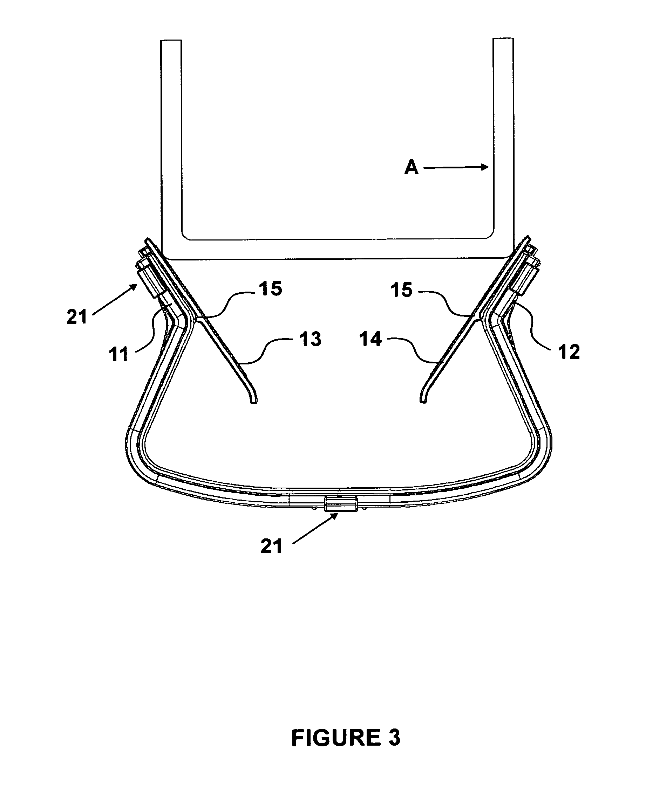

Alternatively, each contact leg 13 or 14 may cover the respective clip end as shown in Figures 3-5.

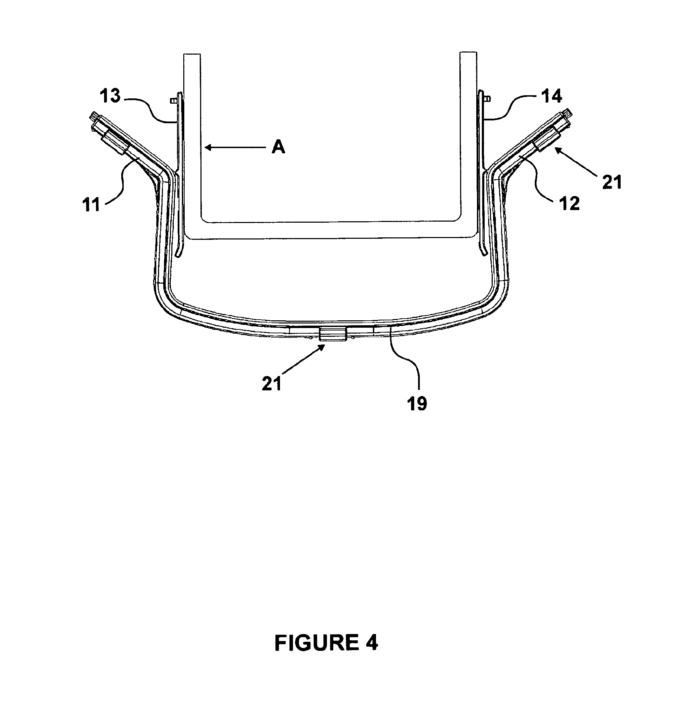

The oppositely directed faces 18 of the clip are formed with grooves 19 which provide detents for drape retention clips 21 as shown in Figures 3 and 4. The drape retention clips 21 are substantially U shaped clips having resilient arms with detents which snap into the opposed grooves 19 on the opposite faces of the mounting clip 10. In use portions of the deformable drape 610 are trapped between the retention clip 21 and mounting clip 210.

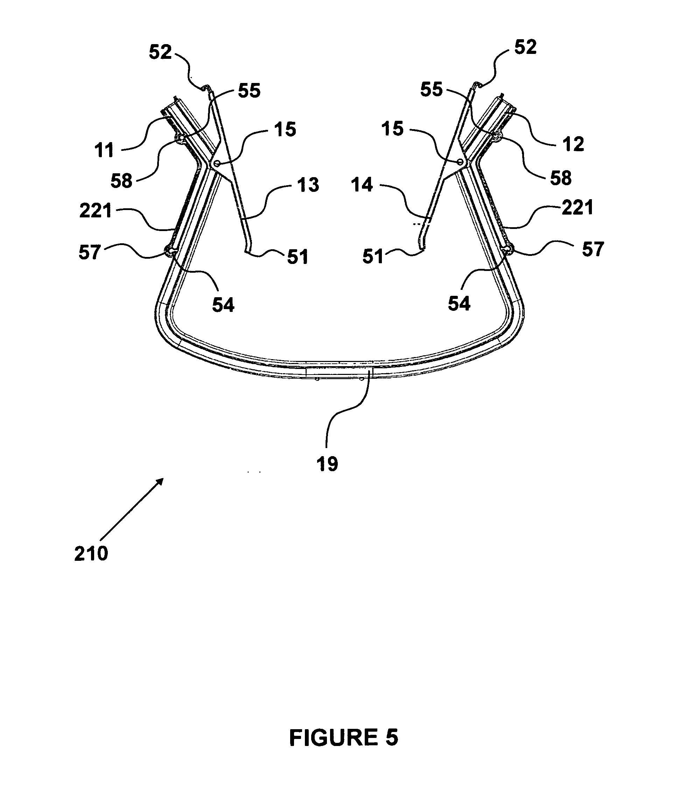

With reference to Figures 3 and 4, there is shown a mounting clip 10 or 210 (as shown in Figure 5) offered up to the arm A such that the contact legs 13 & 14 sit against the surface of the arm A. When the clip is forcibly pressed against the arm, the load on the contact legs 13 & 14 forces the flared ends 11 & 12 apart to open the clip. The clip gradually passing over the arm until the contact point reaches the end of the flared portion. Once the arm passes through the flared portion each leg rotates about its pivot 15 so that the contact legs lie against the sides of the arm as shown in Figure 4. Since the legs are pivoted to the clip they are able to lie with substantially the whole flat surface of each contact leg 13 or 14 against the surface of the arm A giving enhanced grip with the arm A. The pivoted contact leg 13 or 14 is also free to lie against a tapered or inclined surface.

With reference to Figure 5, there is shown another embodiment of the invention in which the mounting clip 210 is substantially similar to the mounting clip 10 and only the differences will be described. The two contact legs 13 & 14 are each provided with an inclined inner portion 51 and the outer end of each contact leg has a shoulder 52 thereon that covers the ends of the clip when the contact legs lie against the flared portions 11 & 12 and serves to hold the contact legs in an open condition.

The drape is held in place on the ends of the mounting clip 210 by a pair of elongate retention clips 221 which extend inwardly from the end of the mounting clip substantially parallel to the flared end portion 11, 12 and slightly beyond. The outer surfaces of the end portions 11 & 12 are provided with spaced apart ribs 54, 55 which are provided with undercuts. The elongate clips 221 are formed with matching grooves 57, 58 which in use cooperate with the ribs 54, 55 to form a resilient snap over connection. Portions of a drape are trapped between the retention clip 221 and the end portions 11, 12 of the mounting clip 210.

With reference to Figure 6, there is shown a cover 110 for sterilizing an arm A of medical equipment, and in particular the C-arms of X- ray machines. The cover includes a drape 610 having dimensions adapted for covering three sides of said C-arm over the whole or part of its length C-arm to cover said three sides of said C-arm and leaves the fourth side of said C-arm uncovered so as not to interfere with the sliding mechanism of the machine. The drape 610 is preferably a readily deformable plastics films such polyethylene, PET, PVC. The cover 110 further comprises a plurality of mounting clips 210 and which are spaced along the cover at desired intervals. The drape is attached to the cover my means of drape retention clips 221 as described earlier. The retention clips trap portions of the drape against the mounting clips 210.

The drape 610 and mounting clips may be sterilized and enclosed in a sterile container ready for use for sterilizing the arm when said cover is attached to the arm.

The drape may be attached to the mounting clips 10/210 by other suitable means including as adhesives, solvent welding, heat welding, and the formation of sealed pockets extending across the cover to accommodate the clips.

It should be understood that particular features relating to the mounting clips and drape retention clips shown in any of the Figures 1 to 5 may be utilised with any of the other mounting clips even though that particular combination is not shown in the drawings, for example the drape retaining clips 221 shown in Figure 5 could be utilised with the mounting clip 10.

## Claims

1. A mounting clip (10, 210) for holding a sterile cover means (110) in place on an arm (A) of a medical apparatus, the mounting clip (10) being a substantially U-shaped clip formed from a resilient material allowing the ends of the clip (11, 12) to flex towards and away from each other to facilitate gripping the arm (A), **characterised in that** the inner end portions of the clip have a contact leg (13, 14) pivoted thereto which in use engages the surface of the arm (A).

2. A mounting clip (10, 210) as claimed in claim 1, wherein the clip has ends (11, 12) flaring outwards for easy attachment to said arm (A), such that the flared ends (11, 12) open out the clip (10) when the clip (10) is forcibly pressed over the arm (A) and snaps on to grip the arm (A).

3. A mounting clip (10, 210) as claimed in claim 2, wherein the contact legs (13, 14) are each pivoted to the clip at the point where the ends of the clips (11, 12) begin the flare outwards.

4. A mounting clip (10, 210) as claimed in claim 3, wherein each contact leg (13, 14) extends on each side of the pivot point (15).

5. A mounting clip (10, 210) as claimed in claim 3 or claim 4, wherein each contact leg (13, 14) extends for substantially the full length of the respective flared end (11, 12).

6. A mounting clip (10, 210) as claimed in claim 5, wherein the outer end of each contact leg (13, 14) is accommodated within a recessed portion of its respective clip end (11, 12).

7. A mounting clip (10, 210) as claimed in claim 5, wherein the outer end of each contact leg (13, 14) extends beyond the respective clip end (11, 12) and has a shoulder (52) thereon which in use covers the respective clip end (11, 12) and serves to hold the respective contact leg (13, 14) in an open condition.

8. A mounting clip (10, 210) as claimed in any of claims 1 to 7, wherein the oppositely directed faces (18) of the clip are formed with grooves (19) therein which provide detents for drape retention clips (21) which engage in the opposing grooves (19).

9. A mounting clip (10, 210) as claimed in any of claims 1 to 8, wherein a drape (610) may be held in place at the ends of the mounting clip (10) by drape retention clips (21) that snap over detents on the outer surface of the flared end portions (11, 12).

10. A mounting clip (10, 210) as claimed in any of claims 1 to 9, wherein said arm (A) of a medical apparatus is a C-arm of an X-ray machine.

11. A cover means (110) for sterilizing an arm (A) of medical equipment, the cover means comprising a drape (610) having dimensions adapted for covering three sides of said arm (A) over the whole or part of its length; and a plurality of mounting clips (10, 210) according to any one of claims 1 to 9 for mounting the cover means (110) to the arm (A) and which are spaced along the cover means (110) at desired intervals.

12. A cover means (110) as claimed in claim 11, wherein said arm (A) of medical equipment is a C-arm of an X-ray machine.

13. A cover means (110) as claimed in claim 11 or claim 12, wherein the drape (610) and mounting clips (10, 210) may be sterilized and enclosed in a sterile container for sterilizing the arm (A) when said cover means (110) is attached to the arm (A).

14. A mounting clip for holding a sterile cover means in place on an arm of a medical apparatus, substantially as described herein with reference to, and as shown in, the accompanying Figures.

15. A cover means for sterilizing an arm of medical equipment substantially as described herein with reference to, and as shown in, the accompanying Figures.
